# EUROPÄISCHE PATENTSCHRIFT

(11) **EP 1 581 374 B1**
(45) Veröffentlichungstag und Bekanntmachung des Hinweises auf die Patenterteilung: **02.08.2006**
(21) Anmeldenummer: 03700006.4
(22) Anmeldetag: 09.01.2003
(51) Int. Cl.: B27B 19/00, F16H 21/18, A61B 17/14, B27B 5/30, A61B 17/16

(54) **VORRICHTUNG ZUR UMWANDLUNG EINER ROTIERENDEN BEWEGUNG IN EINE OSZILLIERENDE BEWEGUNG**
DEVICE FOR TRANSLATING A ROTATIONAL MOTION INTO AN OSCILLATING MOTION
DISPOSITIF POUR TRANSFORMER UN MOUVEMENT DE ROTATION EN UN MOUVEMENT D'OSCILLATION

(43) Veröffentlichungstag der Anmeldung: 05.10.2005
(73) Patentinhaber: Synthes AG Chur, 7002 Chur (CH)
(72) Erfinder: TANNER, Peter, CH-4416 Bubendorf (CH); SCHMUCKLI, Nils, CH-4456 Tenniken (CH)
(74) Vertreter: Lusuardi, Werther
(86) Internationale Anmeldenummer: PCT/CH2003/000006
(87) Internationale Veröffentlichungsnummer: WO 2004/062863

(56) Entgegenhaltungen:
- EP-A- 1 175 870
- DE-A- 1 491 219
- DE-A- 4 425 456
- US-A- 3 103 069
- US-A- 4 069 824
- US-A- 4 281 457
- US-A- 4 509 511
- US-A- 5 658 304
- PATENT ABSTRACTS OF JAPAN vol. 2002, no. 08, 5. August 2002 (2002-08-05) -& JP 2002 102235 A (BRISTOL-MYERS SQUIBB KK), 9. April 2002 (2002-04-09)

## Beschreibung

Die Erfindung bezieht sich auf eine Vorrichtung zur Umwandlung einer rotierenden Bewegung in eine oszillierende Bewegung, gemäss dem Oberbegriff des Patentanspruchs 1

Eine solche Vorrichtung ist aus JP2002 102235 A bekannt. Getriebe, welche rotierende Bewegungen einer Antriebswelle in eine Hin- und Herbewegung wandeln, werden in der Chirurgie seit längerem beispielsweise bei motorbetriebenen Sägen verwendet. Sie können allerdings auch in einer Vielzahl anderer technischer Anwendungen eingesetzt werden.

Ein Getriebe, welches eine rotierende Bewegung einer Antriebswelle in eine Hin- und Herbewegung wandelt ist aus der WO 96/27093 TANNER bekannt. Dieses bekannte Getriebe umfasst einen axial angeordneten Hebel, welcher mittig am Gehäuse um eine senkrecht zur Längsachse stehende Drehachse drehbar gelagert ist, an einem seiner Enden durch einen an der Stirnseite der Antriebswelle exzentrisch angeordneten Nocken oszillierend um die Drehachse angetrieben wird und am anderen seiner Enden über einen weiteren Nocken den Werkzeugträger oszillierend antreibt. Nachteilig an dieser bekannten Vorrichtung ist die axiale Anordnung des Hebels, so dass die Längsachse der oszillierenden Welle rechtwinklig zur Längsachse der Antriebswelle steht.

Hier will die Erfindung Abhilfe schaffen. Der Erfindung liegt die Aufgabe zugrunde, eine Vorrichtung mit einem Getriebe zur Wandlung einer rotierenden Bewegung in eine oszillierende Bewegung zu schaffen, welche eine Anordnung der beiden Wellen mit koaxialen Längsachsen aufweist.

Die Erfindung löst die gestellte Aufgabe mit einer Vorrichtung zur Umwandlung einer rotierenden Bewegung in eine oszillierende Bewegung, welche die Merkmale des Anspruchs 1 aufweist.

Die durch die Erfindung erreichten Vorteile sind im wesentlichen darin zu sehen, dass dank der erfindungsgemässen Vorrichtung eine kompakte Ausführung der Vorrichtung ermöglicht wird, da wegen der koaxialen Wellen der Aussendurchmesser des Gehäuses klein gehalten werden kann.

In der bevorzugten Ausführungsform der erfindungsgemässen Vorrichtung ist der Hebel des Getriebes quer zur Längsachse der Antriebswelle angeordnet.

In einer anderen Ausführungsform der erfindungsgemässen Vorrichtung sind die Antriebswelle und die zweite Welle koaxial zu ihrer Längsachse durchbohrt. Dadurch sind die Vorteile erreichbar, dass
- ein Führungsdraht in die koaxialen Zentralbohrungen einführbar ist; und
- durch Anbringen eines Führungsdrahtes am Knochen und Einführung dieses Führungsdrahtes in diese Zentralbohrungen eine exakte Positionierung sowie eine koaxiale Verschiebung des Sägeblattes am Knochen ermöglicht wird.

In wiederum einer weiteren Ausführungsform der erfindungsgemässen Vorrichtung umfasst die zweite, oszillierende Welle an ihrem hinteren Ende einen exzentrisch zu ihrer Längsachse angeordneten Mitnehmer, welcher mit einer Führung an einem Ende des Hebels in Eingriff bringbar ist. Der Hebel ist am Zapfen rotierbar gelagert und mit einer zweiten Führung am anderen Ende des Hebels an einem festen Nocken am Gehäuse gelagert. Vorzugsweise ist der Hebel mit seinem mittleren Segment am Zapfen rotierbar gelagert. Durch diese Anordnung der Führungen und der Lagerung am Zapfen sind trotz eines kurzen Hebels grosse Drehwinkel für die oszillierende Welle erreichbar.

Der Mitnehmer weist eine Zentralachse auf, welche senkrecht zur Längsachse der zweiten, oszillierenden Welle gemessen einen Abstand a zu dieser aufweist. Ferner weist die Achse des Zapfens senkrecht zur Längsachse der Antriebswelle gemessen eine Exzentrizität e zu dieser auf, wobei das Verhältnis zwischen der Exzentrizität e und dem Abstand a zwischen 0,01 und 0,5 beträgt. Der im Gehäuse fixierte Nocken hat eine Zentralachse, welche senkrecht zur Längsachse der Antriebswelle gemessen einen Abstand b zu dieser aufweist, wobei das Verhältnis zwischen dem Abstand b und dem Abstand a vorzugsweise zwischen 0,5 bis 2,0 beträgt.

In einer bevorzugten Ausführungsform der erfindungsgemässen Sägevorrichtung umfasst diese neben einer Vorrichtung zur Umwandlung einer rotierenden Bewegung in eine oszillierende Bewegung gemäss einer der oben beschriebenen Ausführungsformen ein am vorderen Ende der zweiten Welle lösbar befestigbares Sägeblatt sowie Mittel zur lösbaren Befestigung des Sägeblattes am vorderen Ende der zweiten Welle.

In einer anderen Ausführungsform ist das Sägeblatt als zur Längsachse koaxiales Hohlzylindermantelstück mit einer äusseren Mantelfläche ausgestaltet und weist eine zur Längsachse orthogonale Querschnittsfläche eines Kreisringsegmentes mit einem Zentriwinkel β auf (Hallux-Valgus Sägeblatt). Üblicherweise beträgt der Zentriwinkel β vorzugsweise zwischen 45° und 180°.

In wiederum einer anderen Ausführungsform ist auf der äusseren Mantelfläche des Sägeblattes eine zur Längsachse parallele Skala angebracht, worauf die Tiefe des ausgeführten Sägeschnittes ablesbar ist.

In einer weiteren Ausführungsform umfasst die Sägevorrichtung zusätzlich einen durch die Zentralbohrungen der Antriebswelle und der zweiten Welle durchführbaren Führungsdraht. Das mit dieser Ausführungsform ausführbare Verfahren ist durch die folgenden Verfahrensschritte gekennzeichnet:
a) setzen des Führungsdrahtes an einem Knochen oder Knochenfragment derart, dass die Zentralachse des Führungsdrahtes das Zentrum des auszuführenden, kreisbogenförmigen Sägeschnittes durchstösst;
b) einführen des Führungsdrahtes in die Zentralbohrungen der Antriebswelle und der zweiten Welle der oben beschriebenen Vorrichtung;
c) verschieben der Vorrichtung auf dem Führungsdraht koaxial zur Zentralachse des Führungsdrahtes bis das vordere Ende des Sägeblattes die Oberfläche des zu bearbeitenden Knochens oder Knochenfragmentes berührt; und
d) ausführen des Sägeschnittes durch weiteres axiales Verschieben der Vorrichtung.

In wiederum einer weiteren Ausführungsform weist der Führungsdraht von seiner Spitze gemessen bei einer bestimmten Länge einen axialen Anschlag auf, wodurch die axiale Bewegung der zweiten Welle gegen die Spitze des Führungsdrahtes und damit die Tiefe des Sägeschnittes begrenzt wird.

In einer anderen Ausführungsform umfasst die zweite Welle an ihrem vorderen Ende Mittel zur lösbaren Befestigung des Sägeblattes. Diese Mittel können als Bajonettverschluss oder durch Schraubverbindungen zur lösbaren Befestigung des Sägeblattes ausgestaltet sein.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Ausführungsform der erfindungsgemässen Vorrichtung;
Fig. 2 einen Schnitt A-A durch die in Fig. 1 dargestellte Ausführungsform der erfindungsgemässen Vorrichtung;
Fig. 3a einen zur Längsachse senkrechten Schnitt durch eine Ausführungsform der erfindungsgemässen Vorrichtung, wobei die Antriebswelle in ihrer Ausgangslage ist, d.h. einen Drehwinkel von 0° aufweist;
Fig. 3b einen zur Längsachse senkrechten Schnitt durch die in Fig. 3a dargestellte Ausführungsform der erfindungsgemässen Vorrichtung, wobei die Antriebswelle einen Drehwinkel von 90° aufweist;
Fig. 3c einen zur Längsachse senkrechten Schnitt durch die in den Fig. 3a und 3b dargestellte Ausführungsform der erfindungsgemässen Vorrichtung, wobei die Antriebswelle einen Drehwinkel von 180° aufweist;
Fig. 3d einen zur Längsachse senkrechten Schnitt durch die in den Fig. 3a bis 3c dargestellte Ausführungsform der erfindungsgemässen Vorrichtung, wobei die Antriebswelle einen Drehwinkel von 270° aufweist; und
Fig. 4 eine perspektivische Ansicht einer Ausführungsform der erfindungsgemässen Sägevorrichtung.

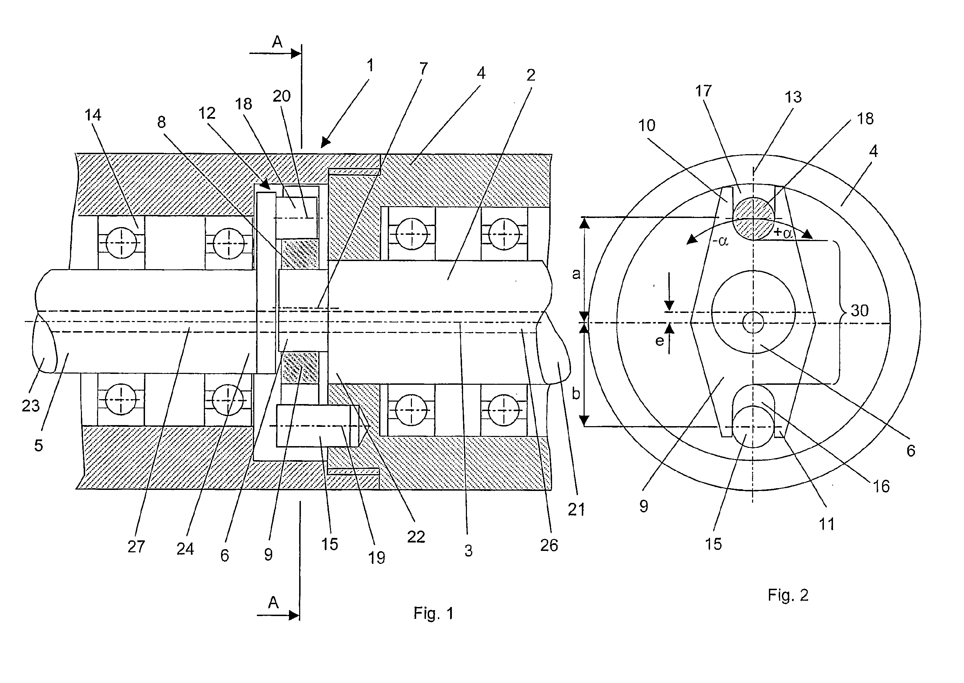

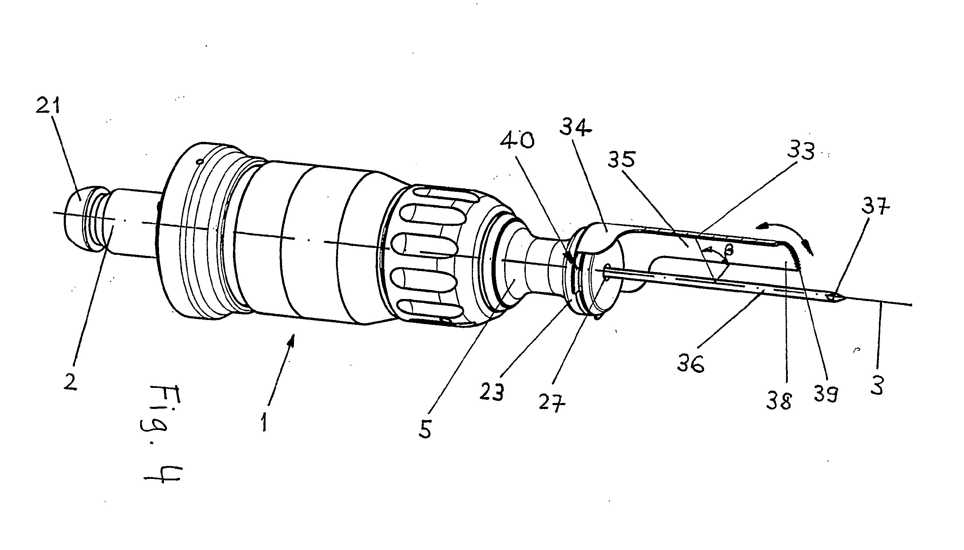

Die in Fig. 1 und 2 dargestellte Ausführungsform der erfindungsgemässen Vorrichtung umfasst im wesentlichen eine um die Längsachse 3 rotierende, mit einem Antriebsaggregat (nicht gezeichnet) verbindbare Antriebswelle 2, eine zweite, endständig mit einem Sägeblatt (Fig. 4) verbindbare Welle, welche zur Längsachse 3 der Antriebswelle 2 koaxial angeordnet ist, sowie ein zwischen den beiden Wellen 2;5 angeordnetes Getriebe 1. Die Antriebswelle 2 und die zweite Welle 5 sind koaxial zur Längsachse 3 in einem mehrteiligen Gehäuse 4 mittels Wälzlagern 14 um die Längsachse 3 rotierbar und axial fest gelagert. Eine Lagerung einer oder beider Wellen 2;5 im Gehäuse 4 mittels Gleitlagern anstelle der Wälzlager 14 ist auch möglich. Durch das Getriebe 1 wird die Rotationsbewegung der Antriebswelle 2 in die rotativ oszillierende Bewegung der zweiten Welle 5 transformiert.

Das Getriebe 1 umfasst einen quer zur Längsachse 3 angeordneten longitudinalen Hebel 9 mit einer Zentralachse 13, einem mittleren Segment 30 und zwei Enden 10; 11, welcher in seinem mittleren Segment 30 mit einem getriebeseitig endständig und exzentrisch an der Antriebswelle 2 angeordneten Zapfen 6 um die Achse 7 des Zapfens 6 rotierbar mit diesem verbunden ist, einen ersten, fest am Gehäuse 4 fixierten Nocken 15 und einen fest mit der zweiten Welle 5 verbundenen Mitnehmer 12, dessen Zentralachse 19 senkrecht zur Längsachse 3 einen Abstand a zu dieser aufweist.

Ferner ist der erste Nocken 15 fest mit dem Gehäuse 4 verbunden und weist rechtwinklig zur Längsachse 3 gemessen einen Abstand b zu dieser auf. Die Enden 10;11 des Hebels 9 sind gabelförmig mit je einer endständig offenen und parallel zur Zentralachse 13 des Hebels 9 angeordneten longitudinalen Führung 16;17. Die erste Führung 16 ist am festen Nocken 15 parallel zur Zentralachse 13 des Hebels 9 verschiebbar und um die Zentralachse 19 rotierbar gelagert. Ferner ist der Mitnehmer 12 mit einem zweiten Nocken 18 ausgestattet. Die zweite Führung 17 ist am zweiten Nocken 18 parallel zur Zentralachse 13 des Hebels 9 verschiebbar und um die Zentralachse 20 des zweiten Nockens 18 rotierbar gelagert. Der Zapfen 6 weist rechtwinklig zur Längsachse 3 gemessen eine Exzentrizität e auf und ist um die Achse 7 des Zapfens 6 rotierbar in der parallel zur Längsachse 3 und senkrecht zur Zentralachse 13 des Hebels 9 angeordneten Zentralbohrung 8 des Hebels 9 gelagert. Bei einer Rotation der Antriebswelle 2 wird der Hebel 9 durch den exzentrischen Zapfen 6 bewegt. Durch die verschiebbare Aufnahme des ersten Nockens 15 in der zweiten Führung 17 am zweiten Ende 11 des Hebels 9 wird der Hebel 9 so geführt, dass die Zentralachse 13 des Hebels 9 die Zentralachse 19 des zweiten Nockens 18 immer schneidet. Die Bewegung des Hebels 9 ist somit eine Überlagerung aus einer Verschiebung parallel zur Zentralachse 13 des Hebels 9 und einer rotativen Oszillationsbewegung um die Zentralachse 19 des zweiten Nockens 18. Dadurch wird die Zentralachse 20 des zweiten Nockens 18 mit einem Drehwinkel von ± α bewegt, wobei der Drehwinkel α abhängig von den Abmessungen a, b und e ist und in einem Bereich zwischen 0,05° und 10° liegt.

Ferner sind die Antriebswelle 2 inklusive Zapfen 6 sowie die zweite, oszillierende Welle 5 mit je einer zur Längsachse 3 koaxialen, durchgehenden Zentralbohrung 26;27 versehen, so dass ein vorgängig an einem Knochen fixierter Führungsdraht in die Zentralbohrungen 26;27 einführbar ist, wodurch die Vorrichtung für den auszuführenden Arbeitsvorgang, beispielsweise Sägevorgang exakt positionierbar ist.

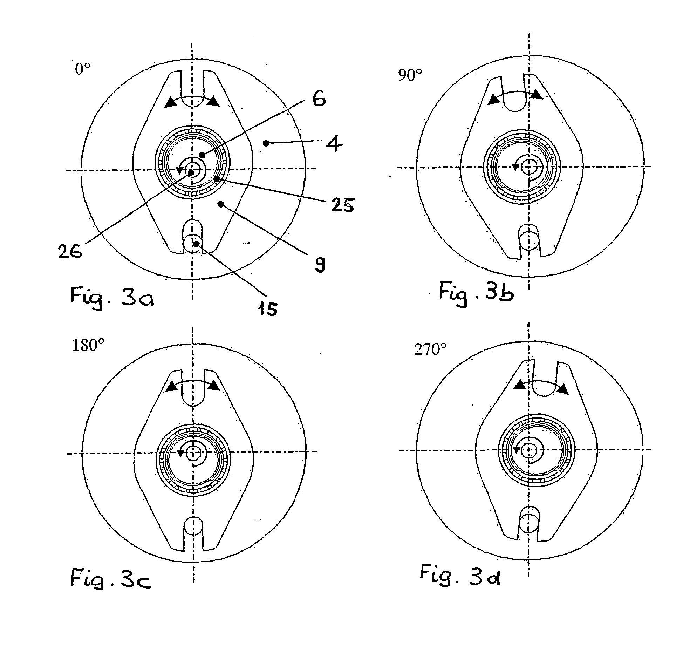

Die in den Fig. 3a bis 3d dargestellte Ausführungsform der erfindungsgemässen Vorrichtung unterscheidet sich von der in den Fig. 1 und 2 dargestellten nur darin, dass der Hebel 9 mittels eines in der Zentralbohrung 8 des Hebels 9 (Fig. 1) eingeführten Wälzlagers 25 drehbar am Zapfen 6 gelagert ist. Ferner ist in Fig. 3a die Antriebswelle 2 in ihrer Ausgangslage, d.h. der Drehwinkel ist 0°. In Fig. 3b ist die Antriebswelle 2 bei einem Drehwinkel von 90° dargestellt, während in Fig. 3c der Drehwinkel der Antriebswelle 2 180°, respektive in Fig. 3d 270° beträgt.

In Fig. 4 ist eine Ausführungsform der erfindungsgemässen Sägevorrichtung dargestellt, welche neben einem Getriebe 1, wie beispielsweise in den Fig. 1 bis 3 gezeigt, ein Sägeblatt 35 und einen Führungsdraht 36 umfasst. Der Führungsdraht 36 ist in die das Getriebe 1 sowie die Antriebswelle 2 und die zweite Welle 5 koaxial durchdringenden Zentralbohrungen 26;27 (Fig. 1) eingeführt, so dass seine Spitze 37 axial über das vordere, mit den Sägezähnen 39 versehene Ende 38 des Sägeblattes 35 hervorsteht. Das Sägeblatt 35 ist als zur Längsachse 3 koaxiales Hohlzylindermantelstück mit einer zur Längsachse 3 orthogonalen, kreisringsegmentförmigen Querschnittsfläche ausgestaltet. Der Zentriwinkel β der kreisringsegmentförmigen Querschnittsfläche beträgt hier ca. 90°. Die Sägezähne 39 sind stirnseitig am vorderen Ende 38 des Sägeblattes 35 angebracht, so dass mit dem Sägeblatt ein zur Längsachse 3 konzentrischer kreisbogenförmiger Sägeschnitt in einem Knochen oder Knochenfragment ausführbar ist. Am vorderen Ende 23 der zweiten Welle 5 sind Mittel 40 zur lösbaren Befestigung des Sägeblattes 35 angebracht, welche beispielsweise als Bajonettverschluss oder Gewindeverbindung ausgestaltet sein können. Durch einen am hinteren Ende 21 der Antriebswelle 2 ankuppelbaren Antriebsmotor (nicht gezeichnet) wird die Antriebswelle 2 des Getriebes 1 angetrieben. Durch das Getriebe 1 wird in der Folge die Rotationsbewegung der Antriebswelle 2 in eine oszillierende Bewegung umgesetzt, so dass das am vorderen Ende 23 der zweiten Welle 5 angeordnete Sägeblatt 35 eine zur Längsachse 3 konzentrische oszillierende Drehbewegung ausführt. Ferner ist auf der äusseren Mantelfläche 34 des Sägeblattes 35 eine zur Längsachse 3 parallele Skala 33 angebracht, worauf die Tiefe des ausgeführten Sägeschnittes ablesbar ist.

## Patentansprüche

1. Vorrichtung zur Umwandlung einer rotierenden Bewegung in eine rotativ oszillierende Bewegung umfassend
A) eine Antriebswelle (2) mit einer Längsachse (3), einem hinteren mit einem Antriebsaggregat verbindbaren Ende (21), einem vorderen Ende (22) und einem am vorderen Ende (22) exzentrisch angeordneten Zapfen (6) mit einer zur Längsachse (3) parallelen Achse (7);
B) eine zweite, rotativ oszillierende Welle (5) mit einem vorderen, mit einem Werkzeug oder Instrument verbindbaren Ende (23) und einem hinteren Ende (24);
C) ein zwischen dem vorderen Ende (22) der Antriebswelle (2) und dem hinteren Ende (24) der zweiten Welle (5) angeordnetes Getriebe (1) zur Umwandlung der rotativen Bewegung der Antriebswelle (2) in die rotativ oszillierende Bewegung der zweiten Welle (5); und
D) ein Gehäuse (4), in welchem die Antriebswelle (2) und die zweite Welle (5) um die Längsachse (3) rotierbar gelagert sind; wobei
E) das Getriebe (1) einen bewegbaren Hebel (9) umfasst, welcher bewegbar am Mitnehmer (12), am Gehäuse (4) und am Zapfen (6) gelagert ist, wobei
F) der Hebel (9) quer zur Längsachse (3) angeordnet ist,
**dadurch gekennzeichnet, dass**
G) die Antriebswelle (2) und die zweite Welle (5) koaxial zur Längsachse (3) durchbohrt sind.

2. Vorrichtung nach Anspruch 1, **dadurch gekennzeichnet, dass** der Hebel (9) mit seinem mittleren Segment (30) am Zapfen (6) und um die Achse (7) des Zapfens (6) rotierbar gelagert ist.

3. Vorrichtung nach Anspruch 1 oder 2, **dadurch gekennzeichnet, dass** das Gehäuse (4) einen festen Nocken (15) umfasst, woran der Hebel (9) mit einem seiner Enden (11) bewegbar gelagert ist.

4. Vorrichtung nach einem der Ansprüche 1 bis 3, **dadurch gekennzeichnet, dass** der Hebel (9) eine Zentralachse (13) umfasst und dass der Hebel (9) an seinem ersten Ende (10) eine zweite zur Zentralachse (13) parallele, longitudinale Führung (17) zur Aufnahme des Mitnehmers (12) umfasst.

5. Vorrichtung nach einem der Ansprüche 1 bis 4, **dadurch gekennzeichnet, dass** der Hebel (9) eine Zentralachse (13) umfasst und dass der Hebel (9) an seinem zweiten Ende (11) eine erste zur Zentralachse (13) parallele, longitudinale Führung (17) zur Aufnahme des Nockens (15) umfasst.

6. Vorrichtung nach einem der Ansprüche 1 bis 5, **dadurch gekennzeichnet, dass** der Mitnehmer (12) eine Zentralachse (20) hat, welche senkrecht zur Längsachse (3) gemessen einen Abstand a > 0 aufweist.

7. Vorrichtung nach einem der Ansprüche 1 bis 6, **dadurch gekennzeichnet, dass** die Achse (7) des Zapfens (6) senkrecht zur Längsachse (3) gemessen eine Exzentrizität e > 0 zur Längsachse (3) aufweist und das Verhältnis e/a zwischen der Exzentrizität e und dem Abstand a zwischen 0,01 und 0,5 beträgt.

8. Vorrichtung nach einem der Ansprüche 1 bis 7, **dadurch gekennzeichnet, dass** der Nocken (15) eine Zentralachse (19) hat, welche senkrecht zur Längsachse (3) gemessen einen Abstand b > 0 zur Längsachse (3) aufweist und das Verhältnis b/a zwischen dem Abstand b und dem Abstand a zwischen 0,5 bis 2,0 beträgt.

9. Sägevorrichtung mit einer Vorrichtung gemäss einem der Ansprüche 1 bis 8, umfassend
a) ein am vorderen Ende (23) der zweiten Welle (5) lösbar befestigbares Sägeblatt (35); und
b) Mittel (40) zur lösbaren Befestigung des Sägeblattes (35) am vorderen Ende (23) der zweiten Welle (5).

10. Sägevorrichtung nach Anspruch 9, **dadurch gekennzeichnet, dass** das Sägeblatt (35) als zur Längsachse (3) koaxiales Hohlzylindermantelstück mit einer äusseren Mantelfläche (34) ausgestaltet ist und eine zur Längsachse (3) orthogonale Querschnittsfläche eines Kreisringsegmentes mit einem Zentriwinkel β aufweist.

11. Sägevorrichtung nach Anspruch 10, **dadurch gekennzeichnet, dass** der Zentriwinkel β vorzugsweise zwischen 45° und 180° beträgt.

12. Sägevorrichtung nach einem der Ansprüche 9 bis 11, **dadurch gekennzeichnet, dass** auf der äusseren Mantelfläche (34) des Sägeblattes (35) eine zur Längsachse (3) parallele Skala (33) angebracht ist.

13. Sägevorrichtung nach einem der Ansprüche 9 bis 12, **dadurch gekennzeichnet, dass** sie zusätzlich einen durch die Zentralbohrungen (26;27) der Antriebswelle (2) und der zweiten Welle (5) durchführbaren Führungsdraht (36) umfasst.

14. Sägevorrichtung nach Anspruch 13, **dadurch gekennzeichnet, dass** der Führungsdraht (36) von seiner Spitze (37) gemessen bei einer bestimmten Länge einen axialen Anschlag aufweist, welcher die axiale Bewegung der zweiten Welle (5) gegen die Spitze (37) des Führungsdrahtes (36) begrenzt.

15. Sägevorrichtung nach einem der Ansprüche 9 bis 14, **dadurch gekennzeichnet, dass** die zweite Welle (5) an ihrem vorderen Ende (23) Mittel (40) zur lösbaren Befestigung des Sägeblattes (35) umfasst.

## Claims

1. A device for translating a rotational motion into an oscillating motion, comprising
A) a drive shaft (2) with a longitudinal axis (3), a rear end (21) that can be connected to a drive unit, a front end (22) and a spigot (6) with an axis (7) that is parallel to the longitudinal axis (3) and is provided eccentrically on the front end (22),
B) a second, rotationally oscillating shaft (5) with a front end (23) that can be connected with a tool or instrument and a rear end (24),
C) a transmission unit (1) provided between the front end (22) of the drive shaft (2) and the rear end (24) of the second shaft (5) to translate the rotational motion of the drive shaft (2) into the rotationally oscillating motion of the second shaft (5), and
D) a housing (4), in which the drive shaft (2) and the second shaft (5) are rotatably mounted about the longitudinal axis (3), wherein
E) the transmission unit (1) comprises a displaceable lever (9), that is displaceably mounted on the carrier (12), on the housing (4) and on the spigot (6), wherein
F) the lever (9) is provided transversely to the longitudinal axis (3).
**characterised in that**
G) the drive shaft (2) and the second shaft (5) are bored through coaxially with the longitudinal axis (3).

2. A device according to claim 1, **characterised in that** the lever (9) is mounted with its central segment (30) on the spigot (6) and rotatably about the axis (7) of the spigot (6).

3. A device according to claim 1 or 2, **characterised in that** the housing (4) comprises a fixed cog (15), on which the lever (9) is displaceably mounted with one of its ends (11).

4. A device according to any one of claims 1 to 3, **characterised in that** the lever (9) has a central axis (13) and that on its first end (10) the lever (9) has a second longitudinal guide (17) that is parallel to the central axis (13) to accept the carrier (12).

5. A device according to any one of claims 1 to 4, **characterised in that** the lever (9) has a central axis (13) and that on its second end (11) the lever (9) has a first longitudinal guide (17) that is parallel to the central axis (13) to accept the cog (15).

6. A device according to any one of claims 1 to 5, **characterised in that** the carrier (12) has a central axis (20), that is at a distance of *a*>0, measured perpendicularly to the longitudinal axis (3).

7. A device according to any one of claims 1 to 6, **characterised in that** the axis (7) of the spigot (6) has an eccentricity of e>0 relative to the longitudinal axis (3) measured perpendicularly to the longitudinal axis (3) and the *e*/*a* ratio between the eccentricity *e* and the distance *a* is between 0.01 and 0.5.

8. A device according to any one of claims 1 to 7, **characterised in that** the cog (15) has a central axis (19) that is at a distance of *b*>0 from the longitudinal axis (3), measured perpendicularly to the central axis (3) and the *b*/*a* ratio between the distance *b* and the distance *a* is between 0.5 to 2.0.

9. A sawing device with a device according to any one of claims 1 to 8, comprising
a) a saw blade (35) that can be detachably fastened on the front end (23) of the second shaft (5), and
b) means (40) for the detachable fastening of the saw blade (35) on the front end (23) of the second shaft (5).

10. A sawing device according to claim 9, **characterised in that** the saw blade (35) is constructed as a hollow cylindrical jacket piece with an external jacket surface (34) that is coaxial with the longitudinal axis (3) and has a cross-sectional surface of a circular segment perpendicularly to the longitudinal axis (3) with a central angle of β.

11. A sawing device according to claim 10, **characterised in that** the central angle β is preferably between 45° and 180°.

12. A sawing device according to any one of claims 9 to 11, **characterised in that** on the external cylindrical surface (34) of the saw blade (35) a graduation (33) is applied that is parallel to the longitudinal axis (3).

13. A sawing device according to any one of claims 9 to 12, **characterised in that** it additionally comprises a guide wire (36) that can pass through the central bores (26, 27) of the drive shaft (2) and of the second shaft (5).

14. A sawing device according to claim 13, **characterised in that** the guide wire (36) has an axial stop at a certain distance from its tip (37), that limits the axial movement of the second shaft (5) relative to the tip of the guide wire (36).

15. A sawing device according to any one of claims 9 to 14, **characterised in that** the second shaft (5) comprises on its front end (23) means (40) for the detachable fastening of the saw blade (35).

## Revendications

1. Dispositif pour transformer un mouvement de rotation en un mouvement de rotation oscillatoire, comprenant
A) un arbre d'entraînement (2) avec un axe longitudinal (3), une extrémité arrière (21) pouvant être reliée à une unité d'entraînement, une extrémité avant (22) et un tourillon (6) disposé de manière excentrique sur l'extrémité avant (22) avec un axe (7) parallèle à l'axe longitudinal (3) ;
B) un deuxième arbre à rotation oscillatoire (5) avec une extrémité avant (23) pouvant être reliée à un outil ou à un instrument et une extrémité arrière (24) ;
C) un mécanisme d'entraînement (1) disposé entre l'extrémité avant (22) de l'arbre d'entraînement (2) et l'extrémité arrière (24) du deuxième arbre (5) pour transformer le mouvement de rotation de l'arbre d'entraînement (2) en le mouvement de rotation oscillatoire du deuxième arbre (5) ; et
D) un boîtier (4), dans lequel l'arbre d'entraînement (2) et le deuxième arbre (5) sont logés de manière à pouvoir tourner autour de l'axe longitudinal (3) ; dans lequel
E) le mécanisme d'entraînement (1) comprend un levier mobile (9) qui est logé de manière mobile sur la manivelle (12), sur le boîtier (4) et sur le tourillon (6), dans lequel
F) le levier (9) est disposé transversalement à l'axe longitudinal (3),
**caractérisé en ce que**
G) l'arbre d'entraînement (2) et le deuxième arbre (5) sont transpercés coaxialement à l'axe longitudinal (3).

2. Dispositif selon la revendication 1, **caractérisé en ce que** le levier (9) est logé sur le tourillon (6) avec son segment central (30) et de manière à pouvoir tourner autour de l'axe (7) du tourillon (6).

3. Dispositif selon la revendication 1 ou 2, **caractérisé en ce que** le boîtier (4) comprend un ergot fixe (15), sur lequel le levier (9) est logé de manière mobile avec une de ses extrémités (11).

4. Dispositif selon l'une quelconque des revendications 1 à 3, **caractérisé en ce que** le levier (9) comprend un axe central (13) et **en ce que** le levier (9) comprend, sur sa première extrémité (10), un deuxième guidage longitudinal (17) parallèle à l'axe central (13) pour recevoir la manivelle (12).

5. Dispositif selon l'une quelconque des revendications 1 à 4, **caractérisé en ce que** le levier (9) comprend un axe central (13) et **en ce que** le levier (9) comprend, sur sa deuxième extrémité (11), un premier guidage longitudinal (17) parallèle à l'axe central (13) pour recevoir l'ergot (15).

6. Dispositif selon l'une quelconque des revendications 1 à 5, **caractérisé en ce que** la manivelle (12) a un axe central (20) qui présente une distance a > 0, mesurée perpendiculairement à l'axe longitudinal (3).

7. Dispositif selon l'une quelconque des revendications 1 à 6, **caractérisé en ce que** l'axe (7) du tourillon (6) présente une excentricité e > 0 par rapport à l'axe longitudinal (3), mesurée perpendiculairement à l'axe longitudinal (3), et **en ce que** le rapport e/a de l'excentricité e à la distance a est compris entre 0,01 et 0,5.

8. Dispositif selon l'une quelconque des revendications 1 à 7, **caractérisé en ce que** l'ergot (15) a un axe central (19), lequel présente une distance b > 0 par rapport à l'axe longitudinal (3), mesurée perpendiculairement à l'axe longitudinal (3), et **en ce que** le rapport b/a de la distance b à la distance a est compris entre 0,5 et 2,0.

9. Dispositif de sciage comprenant un dispositif selon l'une quelconque des revendications 1 à 8, comprenant
a) une lame de scie (35) pouvant être montée de manière amovible sur l'extrémité avant (23) du deuxième arbre (5) ; et
b) un moyen (40) pour monter de manière amovible la lame de scie (35) sur l'extrémité avant (23) du deuxième arbre (5).

10. Dispositif de sciage selon la revendication 9, **caractérisé en ce que** la lame de scie (35) est réalisée sous forme d'enveloppe de cylindre creux coaxiale à l'axe longitudinal (3) avec une surface d'enveloppe externe (34), et présente une section transversale d'un segment annulaire orthogonale à l'axe longitudinal (3) avec un angle au centre β.

11. Dispositif de sciage selon la revendication 10, **caractérisé en ce que** l'angle au centre β est compris de préférence entre 45° et 180°.

12. Dispositif de sciage selon l'une quelconque des revendications 9 à 11, **caractérisé en ce qu'**une graduation (33) parallèle à l'axe longitudinal (3) est réalisée sur la surface d'enveloppe externe (34) de la lame de scie (35).

13. Dispositif de sciage selon l'une quelconque des revendications 9 à 12, **caractérisé en ce qu'**il comprend en plus un fil de guidage (36) pouvant être inséré dans les alésages centraux (26 ; 27) de l'arbre d'entraînement (2) et du deuxième arbre (5).

14. Dispositif de sciage selon la revendication 13, **caractérisé en ce que** le fil de guidage (36) présente une butée axiale placée à une certaine distance de sa pointe (37), laquelle butée limite le déplacement axial du deuxième arbre (5) en direction de la pointe (37) du fil de guidage (36).

15. Dispositif de sciage selon l'une quelconque des revendications 9 à 14, **caractérisé en ce que** le deuxième arbre (5) comprend, au niveau de son extrémité avant (23), un moyen (40) pour monter de manière amovible la lame de scie (35).
